# EUROPEAN PATENT SPECIFICATION

(11) **EP 3 360 595 B1**
(45) Date of publication and mention of the grant of the patent: **03.07.2019**
(21) Application number: 16852977.4
(22) Date of filing: 15.04.2016
(51) Int. Cl.: A61M 16/20, A61M 16/08

(54) **RESPIRATORY VALVE FOR VENTILATOR**
RESPIRATORISCHES VENTIL FÜR VENTILATOR
VALVE RESPIRATOIRE POUR VENTILATEUR

(30) Priority: 08.10.2015 CN 201510644880
(43) Date of publication of application: 15.08.2018
(73) Proprietor: Beijing Aeonmed Co., Ltd., Beijing 100070 (CN)
(72) Inventor: MA, Yingdan, Beijing 100070 (CN); SHEN, Youfang, Beijing 100070 (CN)
(74) Representative: Hanna Moore + Curley
(86) International application number: PCT/CN2016/079345
(87) International publication number: WO 2017/059667

(56) References cited:
- EP-A1- 2 153 859
- WO-A1-00/27455
- WO-A1-2014/037534
- CN-A- 101 745 169
- CN-A- 101 766 862
- CN-A- 102 274 567
- CN-A- 102 274 567
- CN-A- 103 182 140
- CN-A- 104 784 794
- CN-U- 203 943 996
- DE-A1-102008 026 321
- US-A- 3 933 171
- US-A- 4 437 461

## Description

### TECHNICAL FIELD

The present invention relates to the technical field of ventilators, and in particular to a respiratory valve for a ventilator.

### BACKGROUND OF THE INVENTION

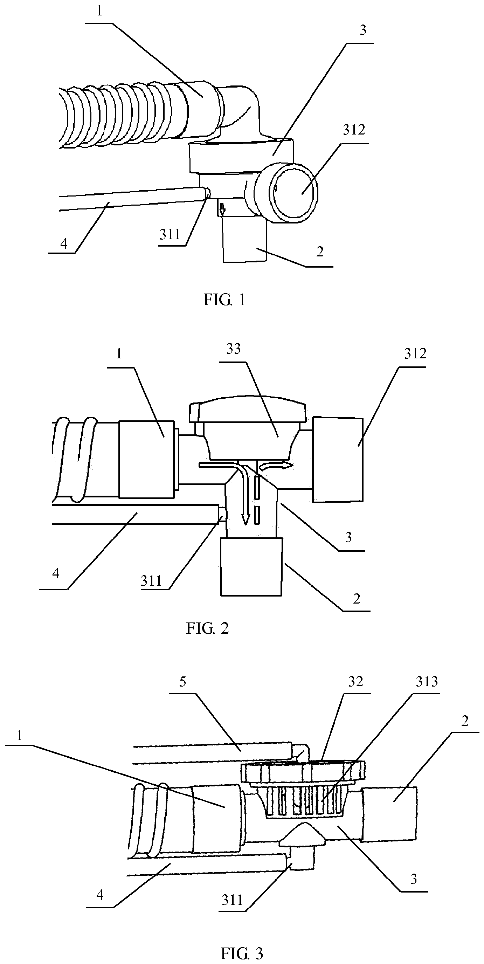

At present, ventilators' control over PEEP has two main manners. In one manner, a mechanical PEEP valve is externally connected at an exhalation port of a respiratory valve, a typical structure of which is as shown in Figs. 1 and 2. As depicted, the mechanical PEEP valve is mounted at a mechanical PEEP port 312 on a respiratory valve 3, and a valve cover 33 matched with the mechanical PEEP valve is mounted at the top of the respiratory valve 3 for the use of patients to expirate. In the other manner, an airflow with pressure provided by a built-in electronic PEEP valve of the ventilator host can be controlled using a software, to offer control gas with different pressures so as to maintain a respective PEEP value. A typical structure is as shown in Fig. 3, wherein a valve cover 32 matched with the electronic PEEP valve is mounted on the respiratory valve 3, and the valve cover is provided with an exhalation port 313 and in communication with the electronic PEEP valve through a PEEP control gas pipeline 5. For this reason existing respiratory valves may also be divided into two types, that is, a respiratory valve equipped with mechanical PEEP and a respiratory valve equipped with electronic PEEP. As limited by their own structures, each type of respiratory valves is only applicable to the foregoing corresponding type of ventilators and cannot be used universally. Usually a hospital or ventilator manufacturer is stocked with various types of ventilators which, further, are equipped with different respiratory valves, thereby causing confusion. Moreover, since there are a variety of respiratory valves in stock, it is adverse to efficient management and storage.

The relevant state of the art is represented by CN 102274567 A, CN 103182140 A, CN 101745169 A, CN 104784794 A, DE 102008026321 A1 and WO 2014/037534 A1.

### SUMMARY OF THE INVENTION

An object of the present invention is to solve such a technical problem that an existing respiratory valve cannot be used universally for all ventilators due to its structural limitation. The present invention provides a respiratory valve for a ventilator, with which ventilators having different types of PEEP valves can be used universally.

To achieve the above object, the present invention provides a respiratory valve for a ventilator as defined in claim 1. The respiratory valve comprises an inhaling pipeline port, a patient end port, a pressure sampling port and a valve port; the respiratory valve further comprises a dual purpose port; the inhaling pipeline port, the patient end port, the pressure sampling port, the valve port and the dual purpose port are in communication with each other through a first cavity provided at the center of the respiratory valve; the respiratory valve is configured to be selectively used with both a mechanical PEEP valve and a built-in electronic PEEP valve of a ventilator; the respiratory valve also comprising both a first valve cover for using the respiratory valve with the mechanical PEEP valve, and a second valve cover for using the respiratory valve with the built-in electronic PEEP valve of the ventilator, whereby, when the respiratory valve is to be used with the mechanical PEEP valve, the dual purpose port is connected to the mechanical PEEP valve and the valve port is connected to the first valve cover, and when the respiratory valve is to be used with the built-in electronic PEEP valve of the ventilator, the dual purpose port is directly used as an exhalation port and the valve port is connected to the second valve cover.

As a further improvement to the above technical solution, a radial cross section of the inhaling pipeline port is covered with a first one-way membrane thereon, the first one-way membrane being mounted on a first support with an air vent which is provided at the end of the inhaling pipeline port, and used for preventing gas within the first cavity from flowing back into the inhaling pipeline port.

As a further improvement to the above technical solution, a secure inhaling port is further comprised, one end of the secure inhaling port being in communication with the atmosphere, and the other end thereof being in communication with the first cavity. A radial cross section of the secure inhaling port is covered with a second one-way membrane thereon, the second one-way membrane being mounted on a second support with an air vent which is provided at the end of the secure inhaling port, and used for preventing gas within the first cavity from flowing back to the atmosphere through the secure inhaling port.

As a further improvement to the above technical solution, an edge of the secure inhaling port at an end close to the atmosphere is provided with an anti-blocking notch.

As a further improvement to the above technical solution, the valve port faces upwards, the valve cover is fastened above the valve port and isolated from the first cavity by an exhalation membrane covering the valve port, whereby a second cavity is formed. The exhalation membrane moves along a vertical direction under the mutual action of air pressures at both sides, an outer wall of the valve port is circumferentially provided with an annular air hole, a bottom of the annular air hole is in communication with the dual purpose port, and gas within the first cavity flows through a gap formed between the exhalation membrane moving upward and the valve port, into the annular air hole, and flows out via the dual purpose port. A control gas channel is provided on a side wall of the inhaling pipeline port, a first annular slot is provided at the end of the control gas channel, a circular hole is provided at an edge of the exhalation membrane, and a lower surface of the circular hole is connected to the first annular slot.

As a further improvement to the above technical solution, an inner surface of the valve cover matched with the mechanical PEEP valve is provided with a second annular slot and a gas-guiding slot, and an upper surface of the circular hole is connected to the second annular slot, so that gas of the inhaling pipeline port flows through the control gas channel, the first annular slot and the circular hole in sequence, to the second annular slot, and gas within the second annular slot is directed to the second cavity by the gas-guiding slot.

As a further improvement to the above technical solution, the valve cover matched with the electronic PEEP valve seals an upper surface of the circular hole, and a top of the valve cover matched with the electronic PEEP valve is provided with an electronic PEEP control port, via which the electronic PEEP valve is connected to the second cavity so that an air pressure within the second cavity is controlled at a set value of the electronic PEEP valve.

As a further improvement to the above technical solution, an outer edge of the annular air hole is circumferentially unevenly distributed with three bosses, and the three bosses are matched with three arc notches provided at an inner surface of the valve cover matched with a respective PEEP valve. An outer surface of the valve cover is provided with a long slot at a position opposite to each arc notch, a protrusion being provided at the middle of one of the long slots for clamping the boss which rotates into the long slot.

A respiratory valve for a ventilator according to the present invention has advantages in that:

the respiratory valve of the present invention enables itself to be used with both a mechanical PEEP valve and an electronic PEEP valve by a dual purpose port provided. When selecting between the mechanical PEEP valve and the electronic PEEP valve, only a valve cover matched with a various PEEP valve needs to be changed to realize a universal usage of different ventilators, thereby increasing an applicable scope and work efficiency of the respiratory valve.

### BRIEF DESCRIPTION OF THE DRAWINGS

FIG. 1 is a structural schematic diagram of an existing respiratory valve which is externally connected with a mechanical PEEP valve.
FIG. 2 is a side cross sectional view of the respiratory valve shown in FIG. 1.
FIG. 3 is a structural schematic diagram of an existing respiratory valve with a built-in electronic PEEP valve.
FIG. 4 is a structural schematic diagram of a respiratory valve equipped with a mechanical PEEP valve according to the present invention.
FIG. 5 is a side cross sectional view of the respiratory valve shown in FIG. 4.
FIG. 6 is a cross sectional view of the respiratory valve shown in FIG. 5 along A-A direction.
FIG. 7 is a structural schematic diagram of a respiratory valve equipped with an electronic PEEP valve according to the present invention.
FIG. 8 is a side cross sectional view of the respiratory valve shown in FIG. 7.
FIG. 9 is a structural schematic diagram of a respiratory valve for a ventilator according to an embodiment of the present invention.
FIG. 10 is a top schematic view of a first valve cover matched with the respiratory valve shown in FIG. 9 according to an embodiment of the present invention.
FIG. 11 is a bottom schematic view of a first valve cover matched with the respiratory valve shown in FIG. 9 according to an embodiment of the present invention.

### Reference numerals

1. inhaling pipeline 2. patient end port 21. outer conical structure 22. inner conical structure 3. respiratory valve 31. valve body 311. pressure sampling port 312. mechanical PEEP port 313. exhalation port 314. dual purpose port 315. inhaling pipeline port 316. first cavity 317. first annular slot 318. control gas channel 319. valve port 32. valve cover matched with an electronic PEEP valve 321. electronic PEEP control port 33. valve cover matched with a mechanical PEEP valve 331. second annular slot 332. gas-guiding slot 34. first one-way membrane 35. exhalation membrane 351. circular hole 36. second cavity 37. secure inhaling port 371. anti-blocking notch 372. supporting edge 38. second one-way membrane 39. second support 4. pressure sampling pipe 5. PEEP control gas pipeline 6. boss 7. arc notch 8. long slot 9. protrusion 10. first support 11. annular air hole

### DETAILED DESCRIPTION OF THE INVENTION

In conjunction with the accompanying drawings and the embodiments, a respiratory valve for a ventilator according to the present invention is below illustrated in detail.

A respiratory valve for a ventilator according to the present invention comprises: an inhaling pipeline port, a patient end port, a pressure sampling port, a valve port and a dual purpose port. The dual purpose port is connected to a mechanical PEEP valve, and the valve port is connected to a valve cover matched with the mechanical PEEP valve, or the dual purpose port is directly used as an exhalation port, and the valve port is connected to a valve cover matched with an electronic PEEP valve.

### Embodiment I

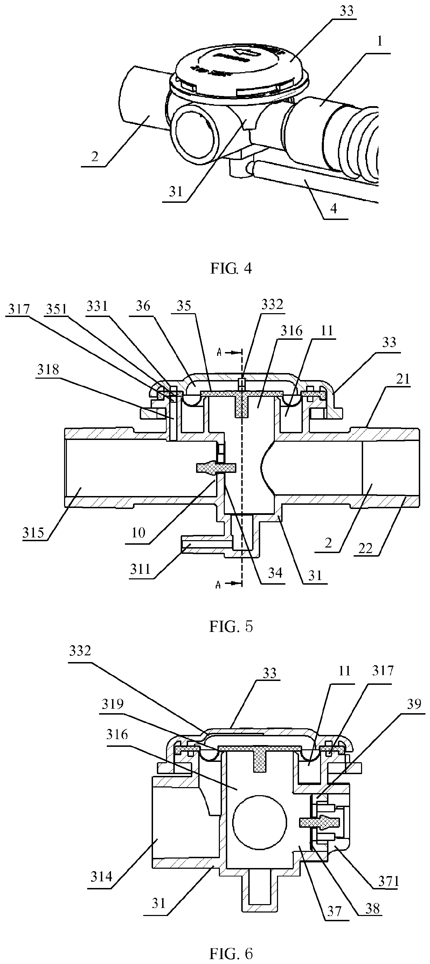

A respiratory valve based on the foregoing structure, such as a respiratory valve equipped with a mechanical PEEP valve as shown in Figs. 5 and 6 specifically comprises: an inhaling pipeline port 315, a patient end port 2, a pressure sampling port 311, a valve port 319 and a dual purpose port 314. The inhaling pipeline port 315, the patient end port 2, the pressure sampling port 311, the valve port 319 and the dual purpose port 314 are in communication with each other through a first cavity 316 provided at the center of the respiratory valve. The dual purpose port 314 is connected to the mechanical PEEP valve, and the valve port 319 is connected to a valve cover 33 matched with the mechanical PEEP valve.

As shown in Fig. 5, a radial cross section of the inhaling pipeline port 315 is covered with a first one-way membrane 34 thereon, and the first one-way membrane 34 is mounted on a first support 10 with an air vent which is provided at the end of the inhaling pipeline port 315, and is used for preventing gas within the first cavity 316 from flowing back into the inhaling pipeline port 315.

The valve port 319 faces upwards, and the valve cover is fastened above the valve port 319 and isolated from the first cavity 316 by an exhalation membrane 35 covering the valve port 319, whereby a second cavity 36 is formed. The exhalation membrane 35 moves along a vertical direction under the mutual action of air pressures at both sides. an outer wall of the valve port 319 is circumferentially provided with an annular air hole 11, and a bottom of the annular air hole 11 is in communication with the dual purpose port 314. Gas within the first cavity 316 flows through a gap formed between the exhalation membrane 35 moving upward and the valve port 319, into the annular air hole 11, and flows out via the dual purpose port 314. A control gas channel 318 is provided on a side wall of the inhaling pipeline port 315, a first annular slot 317 is provided at the end of the control gas channel 318, an edge of the exhalation membrane 35 is provided with a circular hole 351, and a lower surface of the circular hole 351 is connected to the first annular slot 317.

A second annular slot 331 and a gas-guiding slot 332 are provided on an inner surface of the valve cover 33 matched with the mechanical PEEP valve. An upper surface of the circular hole 351 is connected to the second annular slot 331 so that gas of the inhaling pipeline port 315 flows through the control gas channel 318, the first annular slot 317 and the circular hole 351 in sequence, to the second annular slot 331, and gas within the second annular slot 331 is directed to the second cavity 36 by the gas-guiding slot 332.

As shown in Fig. 6, the respiratory valve may further comprise a secure inhaling port 37, one end of the secure inhaling port 37 is in communication with the atmosphere, and the other end thereof is in communication with the first cavity 316. A radial cross section of the secure inhaling port 37 is covered with a second one-way membrane 38 thereon, and the second one-way membrane 38 is mounted on a second support 39 with an air vent which is provided at the end of the secure inhaling port 37, and used for preventing gas within the first cavity 316 from flowing back to the atmosphere through the secure inhaling port 37. An edge of the secure inhaling port 37 at an end close to the atmosphere is provided with an anti-blocking notch 371 for preventing the secure inhaling port 37 from being blocked by a quilt or clothing for negligence.

When a respiratory valve is equipped with a mechanical PEEP valve for use, with reference to the respiratory valve shown in Figs. 4 to 6, the valve cover 33 matched with the mechanical PEEP valve is used with the valve body 31 as a complete set, and the mechanical PEEP valve is mounted on the dual purpose port 314. The inhaling pipeline port 315 is connected to an inhaling pipeline 1, and the pressure sampling port 311 is connected to a pressure sampling pipe 4.

When the ventilator inhales normally, the ventilator supplies a gas mixture of air and oxygen into the first cavity 316 via the inhaling pipeline port 315 and the first one-way membrane 34, wherein the first one-way membrane 34 is mounted on the first support 10 which is provided with an air vent at the end of the inhaling pipeline port 315. A portion of gas within the inhaling pipeline port 315 goes through the control gas channel 318 on the valve body 31, upward through a circular hole 351 on the exhalation membrane 35 and the second annular slot 331 on the valve cover 33 matched with the mechanical PEEP valve, and then through the gas-guiding slot 332 into the second cavity 36. A top of the control gas channel 318 is in communication with the first annular slot 317 at the top of the valve body 31. Such a design may avoid the risk that gas cannot pass through due to non-concentricity of the circular hole 351 of the exhalation membrane 35 and the control gas channel 318. That is, no matter how the exhalation membrane 35 is mounted, the gas mixture of air and oxygen may circulate to the circular hole 351 on the exhalation membrane 35 via the first annular slot 317. The second annular slot 331 provided on the valve cover 33 matched with the mechanical PEEP valve serves the same purpose as the first annular slot 317, i.e. avoiding the risk that the circular hole 315 cannot be connected to the gas-guiding slot 332 when the circular hole 315 deviates from a preset position.

At this point, since a pressure generated by gas in the second cavity 36 on the upper surface of the exhalation membrane 35 is larger than the pressure generated by gas in the first cavity 316 on the lower surface of the exhalation membrane 35, the exhalation membrane 35 is closely attached to the valve port 319 of the valve body 31 to effect a sealing function. In the meantime, the membrane at the secure inhaling port 37 is attached, under the pressure within the first cavity, to the second support 39 which is provided with an air vent at the end of the secure inhaling port 37 to affect a sealing function. A portion of the gas mixture of air and oxygen enters the pressure sampling port 311 to effect gas channel pressure sampling, and the rest of the gas mixture of air and oxygen enters a patient end port 2 to be supplied to a patient. An outer surface of the patient end port 2 is an outer conical structure 21 for connecting with a mask, and an inner surface thereof is an inner conical structure 22 for connecting with a tracheal cannula.

When the ventilator exhales normally, gas exhaled by a patient enters the first cavity 316 via the patient end port 2. At this point, a gas pressure within the inhaling pipeline port 315 is equal to the atmospheric pressure (which is achieved through a control by an internal valve of the ventilator host at the exhalation phase), and in the meantime the second cavity 36 is in communication with the inhaling pipeline port 315 through the gas-guiding slot 332, the second annular slot 331, the circular hole 351 on the exhalation membrane 35 as well as the control gas channel 318, i.e. a gas pressure within the second cavity 36 is also equal to the atmospheric pressure, thus the first one-way membrane 34 is attached, under the pressure of gas in the first cavity 316, to the first support 10 to effect a sealing function so as to prevent the gas exhaled by the patient from flowing back to and contaminating the ventilator via the inhaling pipeline port 315. In addition, under the pressure of gas within the first cavity 316, the exhalation membrane 35 is driven to move upward, at which point a gap appears between the exhalation membrane 35 and the valve port 319. Gas within the first cavity 316 is discharged through this gap, into the dual purpose port 314, and then into the atmosphere via the mechanical PEEP valve.

When a power source of the ventilator is interrupted, there is no gas pressure within the inhaling pipeline port 315. When the patient spontaneously breathes in, a negative pressure may be generated within the first cavity 316, thereby resulting in that the second one-way membrane 38 bends under a force towards the inside of the first cavity 316, and external air enters the first cavity 316 via the air vent on the second support 39 of the secure inhaling port 37 and finally flows into the patient end port 2 for the patient to breathe in. The state in spontaneous expiration is the same as that in normal exhalation of the ventilator.

### Embodiment II

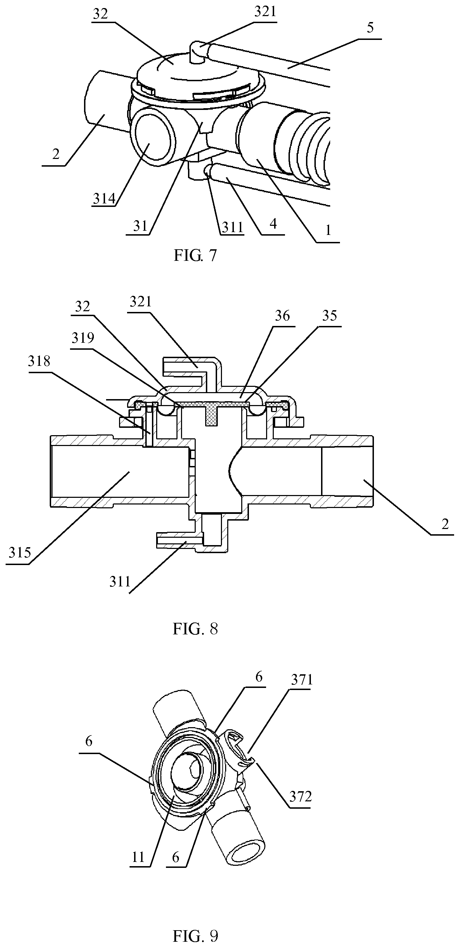

When a respiratory valve is equipped with an electronic PEEP valve for use, with reference to the respiratory valve shown in Figs. 7 and 8, a valve cover 32 matched with the electronic PEEP valve is used with the valve body 31 as a complete set, and the dual purpose port 314 is not connected with any other device but is only used as an exhalation port. If a one-way gas check membrane is provided inside the ventilator host, optionally the first one-way membrane 34 shown in Fig. 5 may not be mounted. The respiratory valve in the present embodiment differs from the respiratory valve in Embodiment I in structure in that, the valve cover 32 matched with the electronic PEEP valve is no longer provided with an annular slot thereon but firmly presses an edge of an exhalation membrane 35 to seal the circular hole 351 on the exhalation membrane 35. Under this state of use, neither the control gas channel 318 on the valve body 31 nor the circular hole 351 on the exhalation membrane 35 takes effect. A top of the valve cover 32 matched with the electronic PEEP valve is provided with an electronic PEEP control port 321, via which the PEEP control gas pipeline 5 is connected to the second cavity 36 so that the gas pressure within the second cavity 36 is controlled at a set value of the electronic PEEP valve.

When the ventilator inhales normally, the pressure on the upper surface of the exhalation membrane 35 comes from the gas pressure given at the electronic PEEP control port 321, and this gas pressure is controlled by the electronic PEEP valve within the ventilator host. At an inhalation phase, the exhalation membrane 35 is closely attached to the valve port 319 of the valve body 31 under a force to effect a sealing function, and the gas mixture of air and oxygen enters the first cavity 316 via the inhaling pipeline port 315 and then flows into the patient end port 2 to be supplied to the patient.

When the ventilator exhales normally, while a gas pressure flowed in at the electronic PEEP control port 321 is reduced to a PEEP set value, and the exhalation membrane 35 is detached from the valve port 319 due to a pressure difference so that a gap is generated, gas within the first cavity 316 is discharged through the gap, into the dual purpose port 314 and then is exhaled.

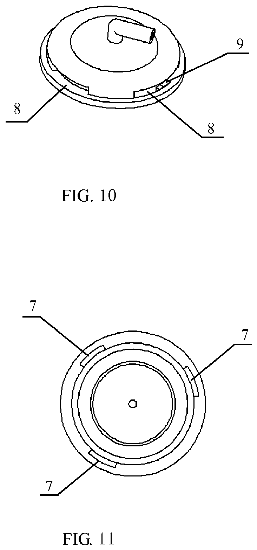

In addition, to guarantee that the pipeline is smooth, the electronic PEEP control port 321 and the inhaling pipeline port 315 are generally rotated to the same direction, which requires the valve body 31 and the valve cover 32 matched with the electronic PEEP valve have a stopping function, so that after the valve cover 32 matched with the electronic PEEP valve rotates in place, the electronic PEEP control port 321 and the inhaling pipeline port 315 are fixed in the same direction. Therefore, with reference to Figs. 9 to 11, a design is made in this embodiment as below:

An outer edge of the annular air hole 11 at the top of the valve body 31 are circumferentially designed with three unevenly distributed and protruded bosses 6, while three arc notches 7 with the same shape are provided at respective positions at the bottom of the valve cover 32 matched with an electronic PEEP. The bosses 6 are matched with the three arc notches 7 provided at an inner surface of the valve cover 32 matched with the electronic PEEP valve. An outer surface of the valve cover 32 matched with the electronic PEEP valve is provided with a long slot 8 at a position opposite to each arc notch 7, whereby three long notches 8 are formed. When mounting, since the bosses 6 and the arc notches 7 are unevenly distributed, after the bosses 6 fall into the arc notches 7, the valve cover 32 matched with the electronic PEEP valve and the valve body 31 can only be mounted in a top-to-down direction due to intervention from other directions. Then, the valve cover is rotated. After the bosses 6 slide into the long slots 8, according to the designed dimension, when the bosses 6 reach the end of the long slots 8, the electronic PEEP control port 321 and the inhaling pipeline port 315 are exactly in the same direction. In addition, a plane where bases of any of the long slots 3 are located may be selected to be designed with a protrusion 9 used for clamping the boss 6 on the valve body to prevent the valve cover 32 matched with the electronic PEEP valve from rotating circumferentially and loosening. Based on the same structural design, the valve cover matched with the mechanical PEEP may be provided thereon with respective arc notches, long slots and protrusions, so that the arc notches are fitted with the bosses and the valve cover matched with the mechanical PEEP is fixed to the valve port by the protrusions.

As shown in Fig. 9, to prevent the secure inhaling port from being blocked by a quilt or clothing for negligence, the secure inhaling port may be provided with the arc-like anti-blocking notch 371. When any clothing blocks the secure inhaling port, a supporting edge 372 without any notch plays a supporting role, so that the anti-blocking notch 371 remains in a suspended state all the time, ensuring that air can smoothly circulate into the respiratory valve.

Finally, it should be explained that the aforementioned embodiments are merely used for illustrating, rather than limiting the technical solutions of the present invention. Although the present invention has been described in detail with reference to the embodiments, those skilled in the art will understand that modifications or equivalent substitutions can be made to the technical solutions of the present invention without departing from the scope of the technical solutions of the present invention, and thereby should all be encompassed within the scope of the claims of the present invention.

## Claims

1. A respiratory valve for a ventilator, comprising: an inhaling pipeline port (315), a patient end port (2), a pressure sampling port (311) and a valve port (319); the respiratory valve further comprises a dual purpose port (314); the inhaling pipeline port (315), the patient end port (2), the pressure sampling port (311), the valve port (319) and the dual purpose port (314) are in communication with each other through a first cavity (316) provided at the center of the respiratory valve; **characterized in that** the respiratory valve is configured to be selectively used with both a mechanical PEEP valve and a built-in electronic PEEP valve of a ventilator; the respiratory valve also comprising both a first valve cover (33) for using the respiratory valve with the mechanical PEEP valve, and a second valve cover (32) for using the respiratory valve with the built-in electronic PEEP valve of the ventilator, whereby, when the respiratory valve is to be used with the mechanical PEEP valve, the dual purpose port (314) is connected to the mechanical PEEP valve, and the valve port (319) is connected to the first valve cover (33), and when the respiratory valve is to be used with the built-in electronic PEEP valve of the ventilator, the dual purpose port (314) is directly used as an exhalation port, and the valve port (319) is connected to the second valve cover (32).

2. The respiratory valve for a ventilator according to claim 1, **characterized in that** a radial cross section of the inhaling pipeline port (315) is covered with a first one-way membrane (34) thereon, the first one-way membrane (34) being mounted on a first support (10) with an air vent which is provided at the end of the inhaling pipeline port (315), and used for preventing gas within the first cavity (316) from flowing back into the inhaling pipeline port (315).

3. The respiratory valve for a ventilator according to claim 1, further comprising a secure inhaling port (37), one end of the secure inhaling port (37) being in communication with the atmosphere, and the other end thereof being in communication with the first cavity (316); and wherein a radial cross section of the secure inhaling port (37) is covered with a second one-way membrane (38) thereon, the second one-way membrane (38) being mounted on a second support (39) with an air vent which is provided at the end of the secure inhaling port (37), and used for preventing gas within the first cavity (316) from flowing back to the atmosphere through the secure inhaling port (37).

4. The respiratory valve for a ventilator according to claim 3, **characterized in that** an edge of the secure inhaling port (37) at an end close to the atmosphere is provided with an anti-blocking notch (371).

5. The respiratory valve for a ventilator according to one of claims 1 to 4, **characterized in that** the valve port (319) faces upwards, the valve cover is fastened above the valve port (319) and isolated from the first cavity (316) by an exhalation membrane (35) covering the valve port (319), whereby a second cavity (36) is formed; and the exhalation membrane (35) moves along a vertical direction under the mutual action of air pressures at both sides, an outer wall of the valve port (319) is circumferentially provided with an annular air hole (11), a bottom of the annular air hole (11) is in communication with the dual purpose port (314), and gas within the first cavity (316) flows through a gap formed between the exhalation membrane (35) moving upward and the valve port (319), into the annular air hole (11), and flows out via the dual purpose port (314); and a control gas channel (318) is provided on a side wall of the inhaling pipeline port (315), a first annular slot (317) is provided at the end of the control gas channel (318), a circular hole (351) is provided at an edge of the exhalation membrane (35), and a lower surface of the circular hole (351) is connected to the first annular slot (317).

6. The respiratory valve for a ventilator according to claim 5, **characterized in that** an inner surface of the valve cover (33) matched with the mechanical PEEP valve is provided with a second annular slot (331) and a gas-guiding slot (332), and an upper surface of the circular hole (351) is connected to the second annular slot (331), so that gas of the inhaling pipeline port (315) flows through the control gas channel (318), the first annular slot (317) and the circular hole (351) in sequence, to the second annular slot (331), and gas within the second annular slot (331) is directed to the second cavity (36) by the gas-guiding slot (332).

7. The respiratory valve for a ventilator according to claim 5, **characterized in that** the valve cover (32) matched with the electronic PEEP valve seals an upper surface of the circular hole (351), and a top of the valve cover (32) matched with the electronic PEEP valve is provided with an electronic PEEP control port (321), via which the electronic PEEP valve is connected to the second cavity (36) so that an air pressure within the second cavity (36) is controlled at a set value of the electronic PEEP valve.

8. The respiratory valve for a ventilator according to claim 7, **characterized in that** an outer edge of the annular air hole (11) is circumferentially unevenly distributed with three bosses (6), and the three bosses (6) are matched with three arc notches (7) provided at an inner surface of the valve cover matched with a respective PEEP valve; and an outer surface of the valve cover is provided with a long slot (8) at a position opposite to each arc notch, a protrusion (9) being provided at the middle of one of the long slots (8) for clamping the boss (6) which rotates into the long slot (8).

## Patentansprüche

1. Atmungsventil für ein Beatmungsgerät, umfassend: einen Einatmungsrohrleitungsanschluss (315), einen Patientenendanschluss (2), einen Druckprobenahmeanschluss (311) und einen Ventilanschluss (319); wobei das Atmungsventil ferner einen Doppelfunktionsanschluss (314) umfasst; der Einatmungsrohrleitungsanschluss (315), der Patientenendanschluss (2), der Druckprobenahmeanschluss (311), der Ventilanschluss (319) und der Doppelfunktionsanschluss (314) miteinander über einen ersten Hohlraum (316), der in der Mitte des Atmungsventils vorgesehen ist, in Verbindung stehen; **dadurch gekennzeichnet, dass** das Atmungsventil ausgelegt ist, selektiv mit sowohl einem mechanischen PEEP-Ventil als auch mit einem eingebauten elektronischen PEEP-Ventil eines Beatmungsgeräts verwendet zu werden; wobei das Atmungsventil auch sowohl eine erste Ventilabdeckung (33) zur Verwendung des Atmungsventils mit dem mechanischen PEEP-Ventil als auch eine zweite Ventilabdeckung (32) zur Verwendung des Atmungsventils mit dem eingebauten elektronischen PEEP-Ventil des Beatmungsgeräts umfasst, wobei, wenn das Atmungsventil mit dem mechanischen PEEP-Ventil zu verwenden ist, der Doppelfunktionsanschluss (314) mit dem mechanischen PEEP-Ventil verbunden ist und der Ventilanschluss (319) mit der ersten Ventilabdeckung (33) verbunden ist, und wenn das Atmungsventil mit dem eingebauten elektronischen PEEP-Ventil des Beatmungsgeräts zu verwenden ist, der Doppelfunktionsanschluss (314) direkt als ein Ausatmungsanschluss verwendet wird und der Ventilanschluss (319) mit der zweiten Ventilabdeckung (32) verbunden ist.

2. Atmungsventil für ein Beatmungsgerät nach Anspruch 1, **dadurch gekennzeichnet, dass** ein radialer Querschnitt des Einatmungsrohrleitungsanschlusses (315) mit einer ersten Einwegmembrane (34) darauf bedeckt ist, wobei die erste Einwegmembrane (34) auf einem ersten Träger (10) mit einer Entlüftung montiert ist, die am Ende des Einatmungsrohrleitungsanschlusses (315) vorgesehen ist und verwendet wird, um zu verhindern, dass Gas innerhalb des ersten Hohlraums (316) zurück in den Einatmungsrohrleitungsanschluss (315) strömt.

3. Atmungsventil für ein Beatmungsgerät nach Anspruch 1, das ferner einen sicheren Einatmungsanschluss (37) umfasst, wobei ein Ende des sicheren Einatmungsanschlusses (37) in Verbindung mit der Atmosphäre steht und das andere Ende davon in Verbindung mit dem ersten Hohlraum (316) steht; und wobei ein radialer Querschnitt des sicheren Einatmungsanschlusses (37) mit einer zweiten Einwegmembrane (38) darauf bedeckt ist, wobei die zweite Einwegmembrane (38) auf einem zweiten Träger (39) mit einer Entlüftung montiert ist, die am Ende des sicheren Einatmungsanschlusses (37) vorgesehen ist und verwendet wird, um zu verhindern, dass Gas innerhalb des ersten Hohlraums (316) durch den sicheren Einatmungsanschluss (37) zurück in die Atmosphäre strömt.

4. Atmungsventil für ein Beatmungsgerät nach Anspruch 3, **dadurch gekennzeichnet, dass** ein Rand des sicheren Einatmungsanschlusses (37) an einem der Atmosphäre nahen Ende mit einer Antiblockierkerbe (371) versehen ist.

5. Atmungsventil für ein Beatmungsgerät nach einem der Ansprüche 1 bis 4, **dadurch gekennzeichnet, dass** der Ventilanschluss (319) nach oben zeigt, die Ventilabdeckung über dem Ventilanschluss (319) befestigt ist und durch eine Ausatmungsmembrane (35) vom ersten Hohlraum (316) isoliert ist, die den Ventilanschluss (319) abdeckt, wodurch ein zweiter Hohlraum (36) gebildet wird; und sich die Ausatmungsmembrane (35) unter der gemeinsamen Wirkung von Luftdrücken auf beiden Seiten entlang einer vertikalen Richtung bewegt, wobei eine Außenwand des Ventilanschlusses (319) umlaufend mit einem ringförmigen Luftloch (11) versehen ist, eine Unterseite des ringförmigen Luftlochs (11) in Verbindung mit dem Doppelfunktionsanschluss (314) steht und Gas im Inneren des ersten Hohlraums (316) durch einen Spalt, der zwischen der sich aufwärts bewegenden Ausatmungsmembrane (35) und dem Ventilanschluss (319) gebildet wird, in das ringförmige Luftloch (11) strömt und über den Doppelfunktionsanschluss (314) ausströmt; und ein Steuergaskanal (318) an einer Seitenwand des Einatmungsrohrleitungsanschlusses (315) vorgesehen ist, ein erster ringförmiger Schlitz (317) am Ende des Steuergaskanals (318) vorgesehen ist, ein kreisförmiges Loch (351) an einem Rand der Ausatmungsmembrane (35) vorgesehen ist und eine Unterseite des kreisförmigen Lochs (351) mit dem ersten ringförmigen Schlitz (317) verbunden ist.

6. Atmungsventil für ein Beatmungsgerät nach Anspruch 5, **dadurch gekennzeichnet, dass** eine Innenfläche der mit dem mechanischen PEEP-Ventil übereingestimmten Ventilabdeckung (33) mit einem zweiten ringförmigen Schlitz (331) und einem Gasführungsschlitz (332) versehen ist und eine Oberseite des kreisförmigen Lochs (351) mit dem zweiten ringförmigen Schlitz (331) verbunden ist, sodass Gas des Einatmungsrohrleitungsanschlusses (315) der Reihe nach durch den Steuergaskanal (318), den ersten ringförmigen Schlitz (317) und das kreisförmige Loch (351) zum zweiten ringförmigen Schlitz (331) strömt und Gas im Inneren des zweiten ringförmigen Schlitzes (331) durch den Gasführungsschlitz (332) zum zweiten Hohlraum (36) gelenkt wird.

7. Atmungsventil für ein Beatmungsgerät nach Anspruch 5, **dadurch gekennzeichnet, dass** die mit dem elektronischen PEEP-Ventil übereingestimmte Ventilabdeckung (32) eine Oberseite des kreisförmigen Lochs (351) abdichtet und ein Oberteil der mit dem elektronischen PEEP-Ventil übereingestimmten Ventilabdeckung (32) mit einem elektronischen PEEP-Steueranschluss (321) versehen ist, über den das elektronische PEEP-Ventil mit dem zweiten Hohlraum (36) verbunden ist, sodass ein Luftdruck im Inneren des zweiten Hohlraums (36) auf einen festgelegten Wert des elektronischen PEEP-Ventils geregelt wird.

8. Atmungsventil für ein Beatmungsgerät nach Anspruch 7, **dadurch gekennzeichnet, dass** ein Außenrand des ringförmigen Luftlochs (11) umlaufend mit drei ungleichmäßig verteilten Vorsprüngen (6) versehen ist und die drei Vorsprünge (6) mit drei bogenförmigen Kerben (7) übereingestimmt sind, die an einer Innenfläche der mit einem jeweiligen PEEP-Ventil übereingestimmten Ventilabdeckung vorgesehen sind; und eine Außenfläche der Ventilabdeckung an einer jeder bogenförmigen Kerbe gegenüberliegenden Position mit einem langen Schlitz (8) versehen ist, wobei ein Überstand (9) in der Mitte eines der langen Schlitze (8) zum Klemmen des Vorsprungs (6) vorgesehen ist, der sich in den langen Schlitz (8) dreht.

## Revendications

1. Valve respiratoire pour un ventilateur, comprenant : un orifice de tube d'inhalation (315), un orifice d'extrémité de patient (2), un orifice d'échantillonnage de pression (311) et un orifice de valve (319) ; la valve respiratoire comprenant en outre un orifice à double fonction (314) ; l'orifice de tube d'inhalation (315), l'orifice d'extrémité de patient (2), l'orifice d'échantillonnage de pression (311), l'orifice de valve (319) et l'orifice à double fonction (314) étant en communication les uns avec les autres à travers une première cavité (316) située au centre de la valve respiratoire ; **caractérisée par le fait que** la valve respiratoire est configurée pour être utilisée sélectivement avec à la fois une valve PEEP mécanique et une valve PEEP électronique intégrée d'un ventilateur ; la valve respiratoire comprenant également un premier couvercle de valve (33) pour utiliser la valve respiratoire avec la valve PEEP mécanique, et un second couvercle de valve (32) pour utiliser la valve respiratoire avec la valve PEEP électronique intégrée du ventilateur, lorsque la valve respiratoire doit être utilisée avec la valve PEEP mécanique, l'orifice à double fonction (314) étant relié à la valve PEEP mécanique, et l'orifice de valve (319) étant relié au premier couvercle de valve (33) et, lorsque la valve respiratoire doit être utilisée avec la valve PEEP électronique intégrée du ventilateur, l'orifice à double fonction (314) étant directement utilisé comme orifice d'expiration, et l'orifice de valve (319) étant relié au second couvercle de valve (32).

2. Valve respiratoire pour un ventilateur selon la revendication 1, **caractérisée par le fait qu'**une section transversale radiale de l'orifice de tube d'inhalation (315) est recouverte avec une première membrane unidirectionnelle (34) sur celle-ci, la première membrane unidirectionnelle (34) étant montée sur un premier support (10) avec un évent qui est situé au niveau de l'extrémité de l'orifice de tube d'inhalation (315), et utilisée pour empêcher un gaz dans la première cavité (316) de s'écouler en retour dans l'orifice de tube d'inhalation (315).

3. Valve respiratoire pour un ventilateur selon la revendication 1, comprenant en outre un orifice d'inhalation fiable (37), une extrémité de l'orifice d'inhalation fiable (37) étant en communication avec l'atmosphère, et l'autre extrémité de celui-ci étant en communication avec la première cavité (316) ; et dans lequel une section transversale radiale de l'orifice d'inhalation fiable (37) est recouverte avec une seconde membrane unidirectionnelle (38) sur celle-ci, la seconde membrane unidirectionnelle (38) étant montée sur un second support (39) avec un évent qui est situé au niveau de l'extrémité de l'orifice d'inhalation fiable (37), et utilisée pour empêcher un gaz dans la première cavité (316) de s'écouler en retour dans l'atmosphère à travers l'orifice d'inhalation fiable (37).

4. Valve respiratoire pour un ventilateur selon la revendication 3, **caractérisée par le fait qu'**un bord de l'orifice d'inhalation fiable (37) au niveau d'une extrémité proche de l'atmosphère comprend une encoche antiblocage (371).

5. Valve respiratoire pour un ventilateur selon l'une des revendications 1 à 4, **caractérisée par le fait que** l'orifice de valve (319) est orienté vers le haut, le couvercle de valve est fixé au-dessus de l'orifice de valve (319) et isolé de la première cavité (316) par une membrane d'expiration (35) recouvrant l'orifice de valve (319), moyennant quoi une seconde cavité (36) est formée ; et la membrane d'expiration (35) se déplace le long d'une direction verticale sous l'action mutuelle de pressions d'air au niveau des deux côtés, une paroi externe de l'orifice de valve (319) comporte, de manière circonférentielle, un trou d'air annulaire (11), un fond du trou d'air annulaire (11) est en communication avec l'orifice à double fonction (314), et un gaz dans la première cavité (316) s'écoule à travers un espace formé entre la membrane d'expiration (35) se déplaçant vers le haut et l'orifice de valve (319), dans le trou d'air annulaire (11), et sort par l'intermédiaire de l'orifice à double fonction (314) ; et un canal de gaz de régulation (318) est prévu sur une paroi latérale de l'orifice de tube d'inhalation (315), une première fente annulaire (317) est située au niveau de l'extrémité du canal de gaz de régulation (318), un trou circulaire (351) est situé au niveau d'un bord de la membrane d'expiration (35), et une surface inférieure du trou circulaire (351) est reliée à la première fente annulaire (317).

6. Valve respiratoire pour un ventilateur selon la revendication 5, **caractérisée par le fait qu'**une surface interne du couvercle de valve (33) mis en correspondance avec la valve PEEP mécanique comprend une seconde fente annulaire (331) et une fente de guidage de gaz (332), et une surface supérieure du trou circulaire (351) est reliée à la seconde fente annulaire (331), de telle sorte qu'un gaz de l'orifice de tube d'inhalation (315) s'écoule à travers le canal de gaz de régulation (318), la première fente annulaire (317) et le trou circulaire (351) en séquence, vers la seconde fente annulaire (331), et un gaz dans la seconde fente annulaire (331) est dirigé vers la seconde cavité (36) par la fente de guidage de gaz (332).

7. Valve respiratoire pour un ventilateur selon la revendication 5, **caractérisée par le fait que** le couvercle de valve (32) mis en correspondance avec la valve PEEP électronique scelle une surface supérieure du trou circulaire (351), et une partie supérieure du couvercle de valve (32) mise en correspondance avec la valve PEEP électronique comprend un orifice de commande PEEP électronique (321), par l'intermédiaire duquel la valve PEEP électronique est reliée à la seconde cavité (36) de telle sorte qu'une pression d'air dans la seconde cavité (36) est régulée à une valeur définie de la valve PEEP électronique.

8. Valve respiratoire pour un ventilateur selon la revendication 7, **caractérisée par le fait qu'**un bord externe du trou d'air annulaire (11) est réparti de manière circonférentielle et irrégulière avec trois bossages (6), et les trois bossages (6) sont mis en correspondance avec trois encoches en arc (7) situées au niveau d'une surface interne du couvercle de valve mis en correspondance avec une valve PEEP respective ; et une surface externe du couvercle de valve comprend une fente longue (8) au niveau d'une position opposée à chaque encoche en arc, une saillie (9) étant prévue au milieu de l'une des fentes longues (8) pour bloquer le bossage (6) qui tourne dans la fente longue (8).
